# EUROPEAN PATENT APPLICATION

(11) **EP 2 700 949 A1**
(43) Date of publication of application: **26.02.2014**
(21) Application number: 12181711.8
(22) Date of filing: 24.08.2012
(51) Int. Cl.: G01N 33/574

(54) **Use of biliverdin reductase proteins as cancer marker**

(71) Applicant: IMG Institut für medizinische Genomforschung Planungsgesellschaft M.B.H., 1010 Vienna (AT)
(72) Inventor: Klocker, Helmut, 6401 Inzing (AT); Schäfer, Georg, 6020 Innsbruck (AT); Seifarth, Christoph, 6020 Innsbruck (AT); Pallua, Johannes D, 6075 Tulfes (AT); Bonn, Günther K, 6170 Zirl (AT); Huck, Christian W, 6020 Innsbruck (AT)
(74) Representative: Sonn & Partner Patentanwälte

(57) **Abstract**

The invention relates to the use of biliverdin reductase proteins as a marker for prostate cancer diagnosis.

## Description

The present invention relates to the detection of markers characteristic of prostate cancer.

Prostate cancer (PCa) is one of the most common malignancies worldwide, the most frequent tumor of males and the second leading cause of cancer death in males in Western countries. PCa is a heterogeneous tumor with individual properties and risk for progression and death with a highly variable natural history. In more than 90% of the cases PCa is a glandular malignant neoplasia (adenocarcinoma) mostly of secretory epithelial cells that line the glands and ducts of the prostate. Development and progression of PCa is a complex multistep process, which is characterized by multiple consecutive mutations that drive the progression of normal cells into highly malignant cancer cells. Cancer cells often show defects in regulatory circuits that govern normal cell proliferation and homeostasis. Diagnosis is based on evaluation of prostate biopsies by a pathologist. Measurement of serum PSA - alone or in combination with digital rectal examination (DRE) - is a widely used tool for early detection of men with high prostate cancer risk. Mostly men with elevated serum PSA and/or suspicious DRE are subjected to prostate biopsy for final diagnosis. However, some men with prostate cancer may have normal levels of PSA and elevated levels of PSA may also be due to other factors, such as age, infarction etc. As a consequence, in 75% of the cases, men with an abnormal DRE and a PSA in this grey zone have a negative, or a seemingly unnecessary biopsy.

Early diagnosis of this disease is still based on the serum test for prostate-specific antigen (PSA), a test with limited disease specificity as a screening tool. Initial diagnosis of PCa involves physical examination, measurement of the concentration of PSA in blood serum and core needle biopsy sampling of prostate tissue. The biopsy material is evaluated histologically and tumors are characterized using the Gleason system - a grading system developed by Donald Gleason based on the histopathological tumor pattern, which is expressed numerically by the Gleason grade (1-5). Since prostate tumors are often multifocal, the Gleason Score (GS) is the sum of the two most prevalent tumor patterns, which are graded 1 (CA1) as the most differentiated to 5 (CA5) as the least differentiated pattern of cancerous gland. This system also describes the aggressiveness of PCa defined by the histologic Gleason Score as a summation of the most and the second most common tumor pattern (Gleason, D.F. Histologic grade, clinical stage, and patient age in prostate cancer. NCI Monogr, 15-18; 1988).

The discrimination between different tumor grades is important with respect to treatment decisions: Currently, many men who are diagnosed with GS 6 prostate cancer are often "over"-treated and risk suffering from urinary and sexual dysfunction - frequent effects of treatment. Therefore, it is also important to develop a sensitive and specific diagnostic tool to distinguish between different tumor grades. The histologically diagnosed Gleason Score indicates tumor dedifferentiation and the potential for tumor progression. Aggressive forms of PCa require aggressive treatment. On the other hand, around one-third of the tumors have a low risk for progression; in these cases a close follow-up surveillance program rather than early aggressive treatment would be appropriate. However, at the time of diagnosis currently no reliable markers are available to distinguish between high and low risk tumors and these results often in overtreatment of these patients.

Biopsy techniques are on a high developed level including ultrasound guidance and optimized techniques (or possibilities) for the visualization of cancer suspicious areas, but a negative result does not exclude the presence of cancer which can be missed by the biopsy procedure. Respectively, the diagnostic biopsy does not represent the most aggressive part of cancer in the whole prostate with insufficient therapy strategies in consequence. This problem is typical for PCa resulting from multi-focality for prostate cancer but also heterogeneity of PCa (with differentially distributed parts of low and high grade cancer in the same specimen) is another significant problem in diagnosis as well as in risk evaluation.

The lack of reliable tools for an exact and rapid diagnosis can easily explain the high interest in the evolution of new techniques to develop new instruments for tumor characterization and risk prediction such as Matrix assisted laser desorption/ionization (MALDI) imaging mass spectrometry (IMS). Due to its unmatched sensitivity and high specificity MALDI IMS is a powerful tool to investigate the spatial distribution of biomarkers within biological samples by in situ analysis of thin tissue sections. MALDI IMS as a functional microscopic imaging can demonstrate the spatial distribution of unlabeled molecules in tissue sections and allows to locate proteins, peptides, lipids, phospholipids, glycolipids, nucleic acids, and metabolites of their mass signals in tissue sections and cell cultures. It can be used to monitor disease specific alterations in situ at the protein level, both qualitatively and semi-quantitatively. This special spatial proteomic imaging method has already demonstrated great promise for the detection and characterization of malignancies in several tissues including lung, breast, brain, ovarian, cervix, colon, pancreas, esophagus (Zhang et al., J Proteome Res 2011; 10(6): 2863-72) and prostate (Cazares et al., Clin Cancer Res 2009; 15: 5541-5551).

Among the most conventional detection methods immunoassays and immunostainings are most common. Specific antibodies are used to detect a certain tumor specific antigen in various samples (e.g. Maines et al., The Journal of Urology 162(4) 1999: 1467). With immunohistological methods it is possible to identify differentially expressed proteins in tissue samples at separate tissue areas. WO 99/18210 A2 discloses compounds and methods for immunotherapy and immunodiagnosis of prostate cancer.

Besides proteomic based approaches, the transcriptome is another cornerstone of modern diagnostics. The evaluation of mRNA levels in a cell is sometimes characteristic for the genetic and epigenetic alterations in a tumor. E.g. Romanuik et al. (BMC Genomics 2009, 10:476) describe the identification of several androgen-responsive genes detected by quantitative real time-PCR. 87 investigated genes were identified or confirmed to be androgen responsive in prostate cancer. Out of these genes, expression of ARL6IP5, CAMK2N1, CENPN, CREB3L4, CXCR7, ERRFI1, FKBP5, KLK3, LRIG1, NCAPD3, PAK1IP1, ST7, and TAOK3 showed marked increase in androgen receptive prostate cancer, being decreased upon androgen deprivation. BLVRB, among others, was one of the genes identified to be androgen responsive but was not differentially expressed in prostate cancer - a result confirmed by the present invention. An advantage of nucleic acid based approaches is the ability for high throughput screenings of differentially transcribed genes, such as described in WO 2005/064009 for colon cancer.

Other diagnostic methods focus on the status of the genome itself, such as identifying the methylation status of marker genes. A methylation marker screening is disclosed in US 2007/0259368.

Although many levels exist to identify diagnostically relevant markers in the diagnosis of a disease, including the genome, transcriptome, or proteome level, interestingly there is little overlap between the markers of each level. Greenbaum et al. (Genome Biology 2003; 4(9):117) have investigated the poor correlation between transcription (mRNA) and protein markers, and suggest the post-transcriptional mechanisms involved in turning mRNA into protein and different in vivo half lives as causes for the observed discrepancy. Similarly, tumor markers vary significantly between cancer types and origins and regrettably for each type need to be identified.

Despite the progress in methodology, there is still a need to provide new diagnostic markers for prostate cancer, which allow an easy and reliable diagnosis of a disease state. It is a goal of the present invention to provide suitable markers and methods to diagnose prostate cancer. This goal is solved by the subject matter of the claims.

According to the present invention, the use of Biliverdin reductase (BVR or BLVR) B and/or A as protein marker for diagnosis of prostate cancer in a tissue sample or in a body fluid sample is provided. Although BVR was identified as transcription (WO 2005/064009) or methylation (US 2007/0259368) markers for colon and gastric cancer, it has been shown that these findings cannot be transferred to other types of cancer. BVR was identified as unsuitable transcription marker in the diagnosis of prostate cancer (Romanuik et al. supra and see example 5). Contrary thereto its protein level surprisingly proved to be a distinctive marker.

BVR has been further identified in renal cell carcinoma (Maines et al., supra), however BVR is not a general cancer marker and no information was known that would allow any conclusion for specific other types of cancer where BVR protein levels might be sufficiently distinctive for a diagnosis of cancer.

Biliverdin reductase (BVR) works within the heme metabolism in the biliverdin/bilirubin redox cycle. It converts biliverdin to bilirubin (a strong antioxidant). The latter is a strong antioxidant protecting from H₂O₂, oxygen and other radicals. In several cellular systems biliverdin reductase was described as a protector from oxidative stress and cellular senescence. Remarkably, billiverdin reductase also has a dual specificity (serin/threonine/tyrosin) kinase activity and seems to have a role in MAP kinase signaling - a major pathway for growth stimulation, which is often deregulated in tumors. Lerner-Marmarosh and colleagues identified billiverdin reductase as a cofactor and nuclear import factor of MAP kinase ERK (Lerner Marmarosh et al., Proc. Natl. Acad. Sci. U. S. A. 2008, 105:6870-6875). Moreover, biliverdin reductase also possesses an SH₂ domain binding site predicted to be a binding site for phosphatidylinositol-3 kinase (PI3K). Like the IRS proteins biliverdine reductase seems to bind to and be phosphorylated by the insulin receptor, thus being an insulin adaptor molecule and involved in metabolic regulation as well. Last but not least, biliverdin reductase is a transcription factor of the leucine zipper protein family containing the characteristic motive of five leucines separated by six other amino acids. It binds to double AP-1 DNA recognition sites and thus may play a role in the AP-1 pathway and it also binds to cAMP response element sites. It enhances the level of activating transcription factor (ATF)-2 and hem oxigenase-1 expression.

US 2011/0217279 proposes administering BVR in treating a PKC-delta related disease, which allegedly includes prostate cancer. No data has been provided by the author to support this allegation. In fact, the same author proposed earlier (US 2010/303790) to use an agent that inhibits BVR (BVR A) in the treatment of allegedly any cancer. Evidently, it has previously to the present invention not yet been established whether an antagonist or agonist - if at all - would be therapeutically effective in the case of prostate cancer.

A second protein with similar function to BVR has been identified, which was subsequently named BVRB or BVR B or BLVRB (UniProt ID P30043). Previously known BVR is therefore nowadays also termed BVR A, BLVRA or still simply BVR (UniProt ID P53004). In general, BVR B is predominant during fetal development, whereas the BVR A dominates in adult life. Although unrelated in sequence they share functional properties and appear to have the same role in prostate cancer and subsequently can contribute to the cancerous phenotypes has been shown herein.

As used herein for describing the invention, "BVR" shall not be limited to BVR A but is used as generic term for both BVR B and BVR A, despite its use in literature wherein it refers to BVR A alone. BVR B is in fact the preferred BVR protein to be used in all embodiments of the invention as it was found more abundantly in prostate cancer. Both, either alone or in combination can be used as inventive protein markers for prostate cancer. Preferably BVR B is determined according to the present invention. In other embodiments BVR A is determined. It is possible to determine both sequentially or simultaneously, such as by using a detection agent such as an antibody being specific for both BVR proteins.

The antibody can be polyclonal for BVR A and/or BVR B and/or be a mixture of antibodies specific for BVR A and BVR B. The detection agents can also be monoclonal antibodies, specific for BVR A and/or BVR B or antibody fragments with such specificity, including Fv, Fab, F(ab') or F(ab) fragments. Suitable derivatives of antibodies include scFvs,² chimeric and humanized antibodies. Also diagnostic monoclonal antibodies from mice can be applied according to the present invention. These are easy and cost effective to be produced in cell cultures and can easily be standardised for routine testing.

The teaching of the present invention is to investigate at least the amount of BVR protein or its degradation products including fragments in the diagnosis, staging, progression, monitoring and/or prognosing PC in a sample of a tumor patient or an individual being at risk or being suspected of having a tumor. BVR is a reliable marker with high specificity and selectivity for cancerous prostate tissue, distinguishing the tumor tissue from stroma or benign prostate tissue.

In a further related aspect, the present invention provides a method for diagnosing prostate cancer in a patient comprising the following steps:
- providing a tissue sample or a body fluid sample of said patient;
- determining the amount of BVR B or BVR A proteins in the sample; and
- comparing the amount of BVR B or BVR A proteins in the sample with a sample of known prostate cancer status.

The invention also provides a method of detecting prostate cancerous cells in a sample of a patient comprising:
- providing a tissue sample or a body fluid sample of said patient;
- determining the amount of BVR proteins in the sample; and
- comparing the amount of BVR proteins in the sample with a sample of known prostate cancer status.

Usually, the amount of BVR protein in the sample is compared with a reference value for this amount with a known PC status, i.e. a value which is known to be a healthy value (i.e. a value not affected by PC) or which is known to be a diseased status with respect to PC, preferably of a given stage of PC. The stage can be determined by Gleason Scoring.

Such reference, standard or control samples are all useable in principle for comparison with the sample of unknown PC status diagnosed according to the present invention. Such reference, standard or control samples can be taken e.g. from a human male subject with negative diagnosis PC or undetectable ongoing PC development. If such a control sample, standard sample or reference sample is said to be comparable to the sample that is taken from a human male subject being suspected to be afflicted by ongoing cancer development according to the present invention, this means that both samples, except for their expression profile, have been derived and treated equally. Thus, the sample in both cases may e.g. be a tissue, urine or blood derived sample which has been further treated in a way to allow for detection of the diagnostic marker molecules as mentioned above.

Although prostate tissue samples from the patient are preferred samples, the present marker is also useable in other suitable samples of tissue or body fluids. For example, the sample may be a blood sample, or a sample derived from blood, such as e.g. a serum sample or a plasma sample or a fraction of a blood, serum or plasma sample. Preferred samples are a saliva sample, a bladder washing sample, a semen sample or a urine sample, especially urine samples after prostate massage. Samples, which do not need prostate biopsies or prostatectomy performed on the patient for providing the samples are specifically preferred for early staging and diagnosis of PC. The control samples of a healthy or known diseased reference might be of the same type as the sample from the patient. The sample of known prostate cancer status is preferably of a healthy subject or a subject with prostate cancer, preferably with a known Gleason Pattern.

The sample of the patient is preferably a sample comprising cells, in particular prostate cells. In non-prostate samples these cells may be disseminated prostate cells. A cellular sample may also be obtained from a patient and BVR be determined in the supernatant of these cells.

The determined amount of BVR may be a protein concentration in a sample. The protein amount may be in a volume or on a surface area of a sample or an amount of BVR per cell in the sample. The amount of BVR may be the combined amount of BVR A and BVR B or BVR A alone or BVR B alone.

The amount of protein present of the markers according to the present invention is measured and is compared with the level of expression of the same marker from other cells or samples. The comparison may be effected in an actual experiment, intellectually (by comparison with known reference values) or virtually (e.g. automatically in silico). When the amount is measurably different, there is according to the invention a meaningful (i.e. statistically significant) difference in the level of expression. Preferably the difference in protein amount of BVR is at least 5%, 10% or 20%, more preferred at least 50% or may even be as high as 75% or 100%. More preferred this difference in the protein amount is at least 200%, i.e. 2-fold, at least 500%, i.e. 5-fold, or at least 1000%, i.e. 10-fold. The BVR amount according to the present invention is higher in a disease sample than in a healthy, normal sample, i.e. at least 5 %, 10% or 20%, more preferred at least 50% or may even be 75% or 100%, i.e. 2-fold higher, preferably at least 10-fold, more preferably at least 50-fold, and most preferably at least 100-fold higher in the disease sample.

According to the present invention the amount of BVR proteins is preferably determined by immunohistochemical (IHC) methods, by immunofluorescence (IF) methods, by mass-spectrometry (MS), by imaging mass spectrometry (IMS) or by a combination of these methods. A preferred MS method is MALDI, especially MALDI-IMS. BVR can be isolated or measured directly on the sample with or without purification. The above mentioned methods have the benefit of providing specific signals without purification from the sample background.

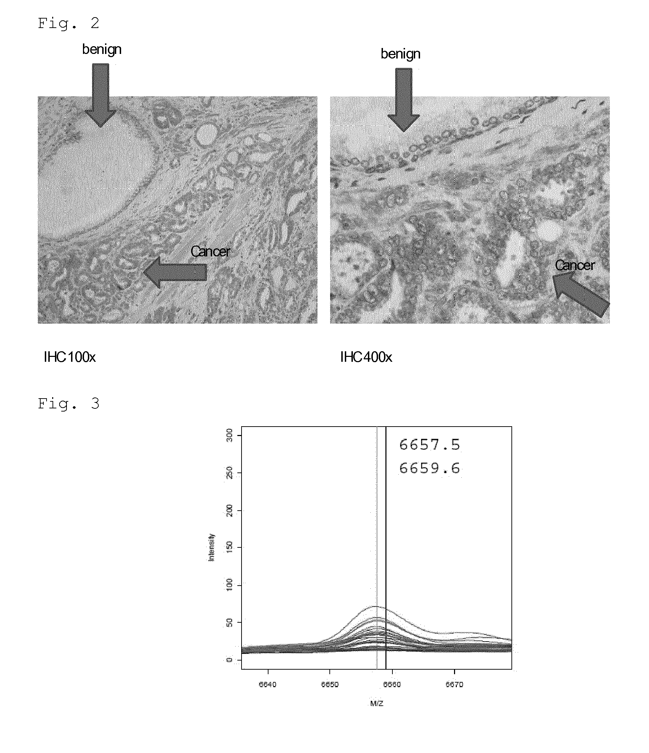

A fragment of BVR B was identified having an m/z ratio of about 6657. Preferably the inventive determination of BVR is by mass spectrometry by determining a signal peak of a protein fragment of BVR A or BVR B. Such a fragment can be detected at an m/z ratio of between 6650 and 6675, preferably of 6657 (s. Fig. 3). A fragment may also be detected at an m/z ratio of 6671 or 6673. According to the invention, the fragment was identified in a fraction containing the fragment corresponding to the m/z peak 6657 of MALDI-IMS (exact m/z 6671,2). Subsequently, differential expression of BVR protein was confirmed by Westernblot.

In preferred embodiments phosphorylated BVR, especially BVR B, is detected. In other embodiments non-phosphorylated BVR is detected.

The core of the present invention is to use BVR as a marker in PC diagnostics. This invention can further be combined with already existing and established markers for PC diagnostics, such as PSA (and/or PSA subtypes), PSM (prostate specific membrane antigen), hK2 (human glandular kallikrein 2), AMACR (alpha-methylacyl-CoA racemase), hepsin or PCA3 (PC antigen 3), among many others.

Any single marker test is prone to error. For example, the standard PSA test is false positive-prone (7 out of 10 men in this category will still not have prostate cancer) and false negative-prone (2.5 out of 10 men with prostate cancer have no elevation in PSA). A combination with BVR according to the invention, e.g. in the determination of a ratio of BVR:second marker, e.g. BVR:PSA, can significantly increase test accuracy.

Thus the invention provides a method of diagnosing prostate cancer in a subject comprising determining BVR, preferably BVR B, in a sample of said subject in combination with determining at least a second non-BVR prostate cancer marker, preferably selected from PSA (and/or PSA subtypes), PSM (prostate specific membrane antigen), hK2 (human glandular kallikrein 2), AMACR (alpha-methylacyl-CoA racemase), hepsin or PCA3 (PC antigen 3).

In fact, although in tissue and urine samples mRNA levels of BVR have not been found to distinguish between cancer and benign conditions, in relation with another non-BVR marker even mRNA levels can be useful to improve diagnostics of -prostate cancer. Such mRNA can even be found in body fluids including urine, where it is secreted as exosomes. Preferably exosomes are isolated and/or purified from the body fluid sample for a determination of BVR (protein or nucleic acids, such as mRNA) according to the invention. Exosomes can be enriched by antibody binding or other immunoaffinity purification methods. Any sample but preferably serum and urine samples are exosome enriched for the inventive method.

The invention further provides a method of controlling a therapeutic intervention in a subject suffering from prostate cancer.

Despite that BVR B and BVR A have ROS mitigating effects that might be of beneficial effect against cancer, surprisingly overexpression of BVR B and -A enhance the oncogenic phenotype of tumor cells, likely through the MAPK pathway (Lerner Marmarosh et al, 2008, supra). This implies that targeting of biliverdin reductase in tumor cells would have a therapeutic effect. Moreover, biliverdin reductase also possesses an SH2 domain binding site predicted to be a binding site for phosphatidylinositol-3 kinase (PI3K). Like the insulin receptor substrate (IRS) proteins biliverdin reductase might be able to bind to and be phosphorylated by the insulin receptor, thus functioning as an insulin adaptor molecule in metabolic regulation as well. Last but not least, biliverdin reductase is a transcription factor of the leucine zipper protein family containing the characteristic motive of five leucines separated by six other amino acids. It binds to double activator protein (AP)-1 DNA recognition sites and thus may play a role in the AP-1 transcription factor pathway. It enhances the level of activating transcription factor (ATF)-2 and heme oxigenase-1 expression. Therefore the present invention provides the inhibition of BVR B or -A or the inhibition of expression of BVR B or -A in the treatment of cancer.

The present invention provides for the first time the significantly increased effects of BVR, especially of BVR B, in cancer and provides the inhibition for the therapy. A BVR, especially BVR B, inhibitor can be provided for use in a prostate cancer therapy. A combination of BVR B and BVR A inhibition can be used.

"Treatment" shall be construed as a beneficial effect on cancer patients, in particular as a reduction of cancer cells, including preventing further progression of cancer, but not necessarily in an absolute curative sense, which is of course possible but not necessarily required by the invention.

Similar, "preventing" shall not be construed as an absolute success to always prevent the onset of cancer, but as a relative reduction of the risk of developing cancer in a patient or of delaying onset of cancer, i.e. as a prophylactic treatment. The treatment, including a prevention, according to the present invention can be used to treat benign tumors or nodules and thus inhibit further development in cancer formation.

A "BVR inhibitor" is to be understood as any compound or agent that reduces BVR activity. It includes any BVR ligand, in particular anti-BVR-antibodies or antibody fragments that bind BVR. In principle the same fragments as described above for diagnostics can be used. Such an antibody or fragment may be adapted for intracellular delivery. Preferably small BVR binding antibody fragments are used that can pass the cell membrane. It is also possible to use delivery formulations such as vesicles.

For antagonizing cellular factors preferably nucleic acid inhibitor molecules are administered to reduce the expression and function of BVR. A nucleic acid inhibitor usually contains an antisense sequence and is capable of hybridizing to the BVR mRNA. Preferred nucleic acid inhibitorsa are antisense inhibitors (DNA or RNA or modified nucleic acids such as LNA) and siRNA. RNA interference (RNAi) is a mechanism to suppress gene expression in a sequence specific manner. RNA interference (RNAi) is a highly effective methodology for suppression of specific gene function in eukaryotic cells. When applied to cells and organisms, RNAi entails the degradation of target mRNA upon transfection of short interfering RNA (siRNA) oligos or short-hairpin RNA (shRNA) encoding vectors. Various methods of RNAi have been described and are generally known for the altering gene expression in plant cells, drosophila and human melanoma cells as is described for example in US 2002/0162126 and US 2002/0173478. The nucleic acid inhibitor for use in the methods and compositions of the invention are selected to target BVR, especially BVR B. In this manner they are targeted to various RNAs corresponding to a target gene. It is understood by one of skill in the art that the nucleic acid inhibitor, including siRNA, as herein described may also include an altered nucleic acid that is a hybrid DNA/RNA construct or any equivalent thereof, doublestranded RNA, microRNA (miRNA), as well as siRNA forms such as siRNA duplications, small hairpin RNA (shRNA) in viral and non-viral vectors and siRNA or shRNA in carriers.

There exist several methods in the art for inhibiting gene expression using RNAi such as described for example in WO 02/055692, WO 02/055693, EP 1 144 623 B1 and WO 03/074654. By using a siRNA therapy any cellular factor can be targeted and inhibited for the inventive BVR antagonizing and inhibiting therapy.

Antisense inhibitors are preferably DNA sequences or modified nucleic acids with increased stability, such as LNA or nucleic acids (both DNA and RNA) with modified backbones such as thiophosphate nucleic acids.

Therefore, any such compound can be used as a BVR inhibitor. A nucleic acid inhibitor may preferably be adapted for intracellular delivery, such as suitable vesicle formulations. An nucleic acid inhibitor encoding vector may also be used in an alternative to nucleic acid inhibitor molecules.

The inhibitor may be used in combination with or for priming a further anti-cancer therapy, preferably a radiation- or chemotherapy. According to this aspect of the present invention a method for increasing the efficacy of cancer therapy in a subject is provided, comprising administering to a subject in need of an effective amount of a BVR inhibitor, wherein said subject is also being administered in the course of a cancer therapy selected from the group consisting of small-molecule drugs, angiogenesis inhibitors, tumor vaccine, chemotherapy, immunotherapy, radiation therapy, gene therapy and combinations thereof.

Another aspect of the present invention is to provide a BVR detection and/or quantification kit for use in PC diagnosis, especially high-risk PC diagnosis. Accordingly, the present invention provides the use of a kit for detecting the amount of BVR in a sample, comprising detection agents for BVR for diagnosing a tissue sample or a sample of a body fluid for PC, especially for distinguishing significant high-risk PC from insignificant low-risk PC.

In general, the kit according to the present invention includes binding agents that specifically bind to BVR protein. These binding agents are preferably linkable or already operably linked to a detectable label. An increased binding of the binding agent specifically bound to BVR protein in the sample of the patient compared to the binding agent bound to BVR protein in a sample of known PC status, e.g. a sample from a healthy individual, indicates the PC status of the patient. In this embodiment of the invention, a medical imaging device or system detects the binding agent specifically bound to BVR. Preferably, the binding agent is immobilised on a solid surface in order to allow simple confirmation of the binding event. Examples for such solid surfaces include microtiter plates, microarrays, ELISA plates, etc.. Exemplary binding agents include natural ligands, synthetic small molecules, chemicals, nucleic acids, peptides, proteins, antibodies and fragments thereof. As already stated above, antibodies against BVR and fragments thereof specifically binding to BVR are preferred binding agents according to the present invention. The inventive kit can be suitable for any of the above described method embodiments. It may comprise antibodies for exosome enrichment.

Exemplary detectable labels include fluorophores, chemical dyes, radioactive compounds, chemiluminescent compounds, magnetic compounds, paramagnetic compounds, enzymes that yield a coloured product, enzymes that yield a chemiluminescent product and enzymes that yield a magnetic product. These labels may be linked to a secondary binding agent, e.g. an antibody specifically recognising the BVR binding agent.

According to a preferred embodiment, the BVR binding agents are detectable by a different label than other markers which are tested, preferably detectable in one and the same assay, e.g. by different fluorophores, different dyes, different colour developing agent systems, etc..

According to a preferred embodiment for determining the amount of BVR in a prostate tissue biopsy sample, BVR and each additional marker can be detected in consecutive tissue slices (preferably about 4-40 µm thickness). Alternatively, a multiple staining can be performed via immunofluorescence.

For IHC staining, a double staining with P63, a marker for basal cells (Weinstein et al., Mod. Pathol. 15 (2002): 1302-1308) is preferred which allows a discrimination between tumor cells and benign areas.

It is evident that any method or use according to the present invention refers to in vitro methods and uses. Samples are provided by usual diagnostic routes and not entailing a substantial health risk.

The present invention is further illustrated by the following figures and examples without being limited to these embodiments of the present invention.

The following abbreviations are used: hematoxylin and eosin (HE), immunohistochemistry (IHC), indium tin oxide (ITO), light microscopy (LM), optimal cutting temperature (OCT), prostate cancer (PCa), prostate-specific antigen (PSA), receiver operating characteristics(ROC), region of interest (ROI), ultrasonography (US), Biliverdin reductase B (BVR B).

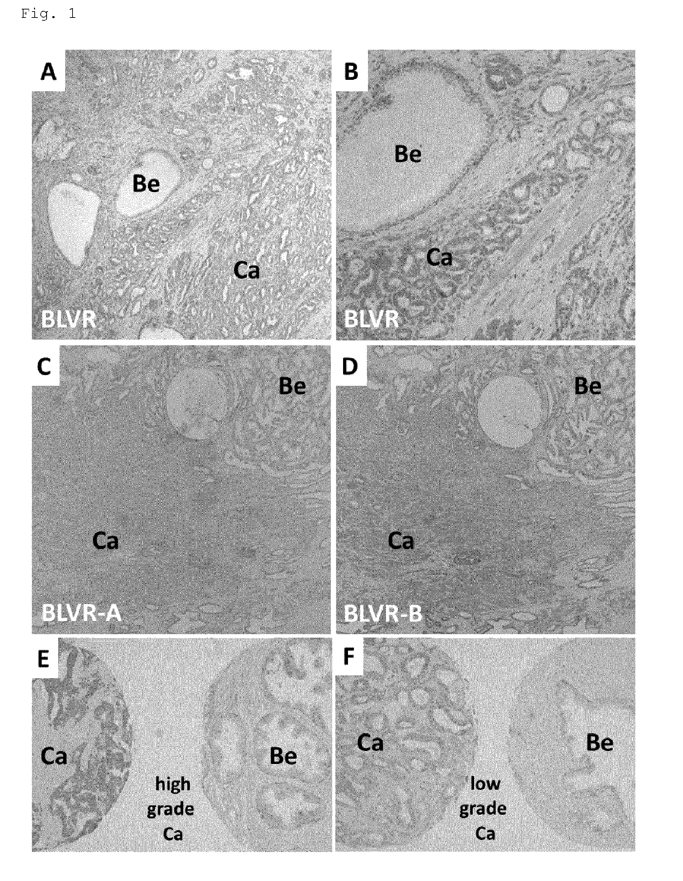

Figure 1 shows an immunohistochemical analysis of biliverdin reductase. Biliverdin reductase stained significantly higher in tumor compared to benign cells (A), (B in higher magnification). Since this antibody was not specified in terms of subtype specificity, IHC of selected cases was repeated with biliverdin reductase A and biliverdin reductase B specific antibodies (C and D). Both BLVR types revealed similar distribution patterns. For statistical analysis of expression a tissue array with almost 100 prostate cancer cases included was stained and immunoreactivity scored and analyzed. Figures (E) and (F) illustrate representative high grade and low grade tumor cases, respectively.
Figure 2 shows immunohistological investigation of prostate tissue. Benign and cancerous areas are marked. Left: 100x magnifications, right: 400x magnification.
Figure 3 shows MALDI Peak at m/z 6657 for various samples. Higher peaks can be observed for cancer samples than for benign samples. Grey vertical line: m/z peaks, black line: area under curve (AUC) maximum.
Figure 4 shows a box plot analysis of the IHC intensities for benign and cancerous tissue samples.
Figure 5 shows an IHC histogram with mean intensities for BVR B in benign (left) and carcinoma tissue (right). Benign: average int. 1.52, sd 0.468, N=65; Cancer: average int. 2.14, sd 0.549, N=94.
Figure 6 shows a combination of BLVRB and PSA marker analysis in urine samples. The differences between benign and PCa groups were of statistical significance.

### Example 1: Materials and Chemicals

Acetonitrile (≥ 99.9 % vol) and ethanol (≥ 96 % vol) were purchased from Sigma Aldrich (St. Louis, MO, USA), OCT (optimum cutting temperature polymer) for embedding tissue from Gatt-Koller (Absam, Austria), Hematoxylin (hematoxylin solution according to Mayer) and Eosin (Eosin Y) from Sigma Aldrich. TFA (for protein sequence analysis 99.5%) and sinapinic acid (matrix substance for MALDI-MS, Ultra pure) were purchased from Fluka (Buchs, Switzerland). The protein calibration standard containing insulin, ubiquitin I, cytochrome C and myoglobin was from Bruker Daltonik Care (Bremen, Germany). ITO coated conductive glass slides (Bruker Daltonik, Bremen, Germany), glass and Superfrost glass slides (Menzel slides, Fisher Scientific, Vienna, Austria) were used for tissue MALDI imaging-MS and histopathological analysis, respectively.

### Example 2: Sample Collection and Tissue Specimens

Prostate tissue was provided by the prostate cancer biobank of the Departments of Urology and Pathology at the Innsbruck Medical University. The specimens were procured from patients giving informed consent and with the approval of the Ethics Committee. 15 patients who underwent radical prostatectomy due to cancer (13 cases) or benign prostatic hyperplasia (BPH) (2 cases) between 2002 and 2009 were recruited for this study. Their age range was 45 to 76 years, with a mean age of 62.7 years. The radical prostatectomy specimens were divided longitudinally to create mirrored cores; one was fixed and paraffin embedded and the other was embedded in OCT, snap frozen in liquid nitrogen (not later than 15 minutes after removal of the prostate), and stored at -80°C. The histomorphological and clinical characteristics of the analyzed tumor cases are listed in Tab. 1. All cases were histopathologically reviewed by an uropathologist according to the American Society of Clinical Oncology/College of American Pathologists guideline recommendations for Gleason grading. Cancer, benign and stroma regions were assigned using HE staining and P63/AMACR double immunostaining. P63 is a marker of basal epithelial cells, which are absent from tumor glands whereas AMACR antibodies stain prostate tumor cells. A tissue microarray (TMA) was used for validation of protein markers by immunohistochemistry and contained tissue cores of 94 radical prostatectomies (tree tumor and one non-malignant benign tissue core for each case). The specimens for construction of this TMA also obtained from the prostate cancer biobank from a patient cohort independent of the cohort used for tissue imaging analysis.

| Tab. 1: Characteristics of patients | | |
|---|---|---|
| Cancer cases | | 13 |
| mean age at diagnosis | | 62.7 (45 to 76) |
| mean PSA value (ng/mL) | | 6.7 (1.94 to 18.09) |
| Grade | | |
| | GS 5 | 2 |
| | GS 6 | 2 |
| | GS 7 | 4 |
| | GS 9 | 5 |
| Stage | | |
| | pT2 | 7 |
| | pT3 | 6 |
| BPH cases | | 2 |
| mean age at diagnosis | | 71.5 |

### Example 3: Assessment of the Gleason grade

All cases were carefully reviewed by an uropathologist and characterized according to the Gleason Classification System for PCa. They were evaluated by immunohistochemistry (IHC) and HE staining to identify cancer, benign and stroma regions within the tissue sections. Scoring criteria were applied according to the American Society of Clinical Oncology/College of American Pathologists guideline recommendations for Gleason grading. A histologically defined PCa area was confirmed by IHC staining using p63 (basal cell marker) with a negative expression in cancer and positive expression in benign.

### Example 4: mRNA profiling

Expression profiling on human tumor and benign prostate tissue samples was performed with an Illumina microarray comprising a probe for BVL-B (Illumina ID 6620521, sequence database entry NM_000713.1, probe sequence GGACATGAGGAGCAAAGGAAGGGGGCAA-TAAATGTTGAGCCAAGAGCTTC (SEQ ID NO: 1)). Analysis was for differentially expressed genes between tumor and benign tissues, and between tumors with higher Gleason pattern (4+3 and higher) and lower Gleason pattern (3+4 and lower). In tumor tissue BVL-B mRNA is slightly, but not significantly, downregulated when compared to benign tissue. It can be concluded that BVL-B mRNA is not suitable to distinguish between healthy and diseased states or between different stages of prostate cancer.

### Example 5: MALDI-MS tissue imaging analysis

Fresh frozen tissue sections were prepared from frozen tissue blocks. Before cryocutting OCT embedding medium was carefully removed and two 4 µm (one for HE and one for P63 immunohistochemical quick staining) were cut (Leica Kryostat CM 3050) and stained for orientation. After histological assignment of tissue types, 12 µm sections were cut for MALDI-MS tissue imaging and mounted onto indium tin oxide (ITO) coated conductive glass slides. The sections were washed two times with 70% ethanol for 1 min followed by two times with absolute ethanol for 1 min and dried in a vacuum desiccator for 15 min. Matrix deposition was performed on an ImagePrep station (Bruker Daltonik, Germany) using the Bruker standard protocol (tissue coating by a matrix solution consisting of 10 mg/ml sinapinic acid in 60% acetonitrile, 0.2% tri-fluoro-acetic acid). The Imaging MS measurements were performed on an UltrafleXtreme instrument (Bruker Daltonik, Germany) in positive linear mode using FlexControl 3.0 and FlexImaging 3.0 software packages (all Bruker Daltonik). Ions were detected in a mass range of m/z 2000 to m/z 20000 using a sampling rate with a lateral resolution of 100 µm. A total of 200 laser shots were accumulated per spot at constant laser power. A ready-made protein standard (Bruker Daltonik, Germany) was employed for calibration of spectra, which was done externally on the same target before each measurement. After the measurements the slides were incubated in 70% ethanol to remove the matrix, then washed in distilled water and HE stained. Slides were then scanned with the MIRAX DESK system (Carl Zeiss MicroImaging GmbH, Göttingen, Germany) and images were co-registered with the MALDI-MS tissue imaging results.

### Example 6: Imaging data analysis and identification of BVR B m/z peaks

In the sections stained after MALDI-MS tissue imaging the regions of interest (ROIs) (benign, stroma and cancer region) were assigned by an uropathologist. Tissue type-associated spectra were selected using the FlexImaging 3.0 software (Bruker Daltonik). Extracted mass spectra of ROIs underwent recalibration on common "background" peaks (spectral alignment) and normalization based on their total ion count in the observation mass range utilizing Clin-ProTools 2.2 software (Bruker Daltonik). An average spectrum and average peak intensities across all spectra of each ROI were calculated. Significant differences in peak intensities between the histological groups were evaluated by the Wilcoxon rank-sum test with a significance cutoff of p>0.05 and principle component analysis. Significant differences in peak intensities between the histological groups were also identified by visual inspection and the best discriminatory m/z peaks were selected.

In a second statistical approach a bioinformatics workflow including preprocessing and feature selection algorithms was used to identify discriminatory biomarker candidates (WO 2008/037479). For the analysis of the raw MALDI IMS spectra a two-step strategy was developed consisting of a data processing modality coupled with a paradigm for the targeted identification of highly discriminating m/z values in the MS spectra (putative candidate biomarkers). The pre-processing pipeline comprised the binning of m/z values, adjustment of m/z values, baseline correction, normalization, peak detection, quality assessment, alignment to internal standards, and generation of mean spectra (when having multiple spectra per subject or image). For the targeted search of highly discriminating m/z values an adjusted version of a feature selection algorithm (Zhu and Hestie, 2004 Biostatistics 2004, 5:427-443) was applied. It delivers a list of potential candidate m/z masses (sorted by their discriminatory ability) as well as a subset of candidates of this list showing the highest discriminatory ability in terms of the area under the receiver operating characteristics (ROC) curve.

### Example 7: Tissue preparation and protein identification

A protein extract prepared from whole tissue cryosections including cancer, non-malignant benign and stroma tissue types was used for protein identification. OCT embedding medium was carefully removed and 6 cryosections of 10 µm thickness (tissue area 1 cm²) were placed in a 1.5 ml Eppendorf tube with 180 µl of ice cold 0.1% formic acid. Tissue lysis was performed by ultrasonication in an ice-cold ultrasound water bath for 15 min followed by freezing and thawing of the suspension. After additional ultrasonication for 15 min, the suspension was shaken for 20 min at 4°C. Subsequently, the extract was cleared by centrifugation at 20000 g for 1.5 h at 2°C. The supernatant was then transferred into a ultrafiltration tube (Vivaspin 500 - 3000 MWCO PES, Sartorius, Goettingen, Germany), followed by centrifugation at 15000 g for 1 h at 2°C. The ultrafiltration tube was washed 3 times with 150 µl 0.1% formic acid and the centrifugation was repeated after each addition of washing solution. Solubilization of the proteins from the ultrafiltration tube was performed with 110 µl 0.1% formic acid on an Eppendorf shaker for 20 min at 4°C. Protein concentration was measured using a Bradford protein assay (2.2 µg/µl) and aliquots were frozen and stored at -20°C until use.

For protein fractionation and identification 20 µg of protein extract was separated on a nanoHPLC-system using a 15 cm/75 µm (length/inner diameter) reversed phase C18 PepMap 100, 3 µm column (Dionex, Sunnyvale, CA). The separation was done using an UltiMate 3000 pump (Dionex) at a flow rate of 250 nL/min using gradient elution (solvent A: 0.1% formic acid in water; solvent B: 85% acetonitrile/0.1% formic acid) starting at 4% B. The concentration of solvent B was maintained at 4% for 2 min, increased linearly to 50% over 50 min and from 50% to 100% over 10 min. The eluent from the HPLC column was mixed post column with matrix solution consisting of 6.0 mg/mL alpha cyano-4-hydroxycinammic acid (CHCA, Fluka, Buchs, Switzerland) and 11.5 nMol/L angiotensin I (internal standard, BACHAM, Switzerland) dissolved in 70% acetonitrile and 0.1% TFA. HPLC-fractions of 15 sec consisting of one part HPLC eluent and two parts of matrix solution were collected with the Probot micro fraction collector and directly spotted across a MALDI plate using a 53 well by 32 well pattern. Mass spectrometric measurements of the collected spots were performed on a 4800 Plus MALDI TOF/TOF Analyzer instrument (Applied Biosystems, Foster City, CA) using the 4000 Series Explorer Software V3.6. for data acquisition and processing. MS spectra were acquired in positive reflector mode (m/z 800 - 4000) and the linear mode (m/z 3000 - 30000) by accumulation of single measurements from 2000 laser shots. For each spot the ten most intense peaks in the MS spectrum were selected as precursor ions for MS/MS measurements using air gas for collision-induced dissociation (CID). Single measurements of 2000 laser shots were accumulated for each MS/MS spectrum. For protein identification the MS/MS data were processed with ProteinPilot 3.0 software (Applied Biosystems) using the NCBI human protein database. For protein identification the following settings were used: Cystein alkylation none, digestion none, false discovery rate (FDR) yes. Precursor tolerance for MS was 0.2 Da and for MS/MS 0.4 Da.

### Example 8: MALDI IMAGING - Protein identification-second run

Prostate tissue extracts were prepared according to Rauser et al. (J Proteome Res. 2010, 9(4):1854-63). Sample 2 tissue protein extract (#1425) was fractionated on a nano-HPLC column, continuously mixed with matrix and online spotted onto a MALDI target. Altogether some 300 fraction spots were investigated. The spotted fractions were then analyzed in MALDI-TOF linear mode to assign the peaks of interest to the analyzed fractions. In a second run using MALDI-TOF-TOF the proteins of the different fractions were fragmented and identified. Several of the candidate m/z masses were identified within a mass range of about ± 15 Da. Biliverdin reductase B (flavin reductase) with a MALDI imaging pattern benign epithelium < tumor cells was selected for further validation by immunohistochemistry: It could be identified in a fraction linked to the tumor-associated m/z mass 6671,2 (spot +/- 15 linear mode), MALDI Imaging peak: 6658.

### Example 9: Protein identification of the MALDI-IMS investigated masses

The protein identification of the biomarker candidates were performed on an amaZon ETD Ion Trap, LTQ Orbitrap XL, Thermo Finnigan and a 4800 Plus MALDI TOF/TOF Analyzer instrument (Applied Biosystems, Foster City, CA). The identification of potential biomarkers identified by MALDI imaging approach is performed by using top-down proteomics. First, tumor cells which were harvested by macrodissection are disrupted by ultrasound on ice in 0.1% TFA. Subsequently, the cell disruption is centrifuged and the obtained supernatant is separated by using a size exclusion chromatography. The fractions are then collected in a 96-well plate and checked with MALDI-TOF MS. Those fractions that have the tumor masses are then analyzed.

The MALDI-IMS peak with a m/z of 6658 was identified as Biliverdin reductase B (BVR B) as a 3 MH+ ion of a fragment.

### Example 10: MS analysis and results

To detect proteomic patterns in different prostate tissues (benign, stroma and cancer), protein profiles of tissue sections of 15 radical prostatectomy specimens were assessed utilizing MALDI IMS. All tissue sections were uniformly coated with the MALDI matrix solution using an automated spraying device. After MALDI IMS, each section was HE stained and its digital histological image was then co-registered with the MALDI IMS results to correlate massspectrometric data with the histological features. This allowed a histology-driven analysis of the mass spectra obtained from the measured tissue samples. Regions of interest containing prostate adenocarcinoma cell populations, predominantly benign epithelial cells or stromal cells were designated. Ions with significant discriminating power were evaluated in the analyzed tissues, and images were derived from the histology-directed ROI, which allowed for a visual determination of cell-type-specific changes in peptide ion expression.

Additionally, principal component analysis (PCA) was used for statistically analyses of different patients. With PCA the dimensionality of MALDI IMS data sets were reduced while as much information as possible was retained. The scores of the first principal components were used to generate meaningful images. For the PCA analysis across the 5 different patients, the mass spectra of the selected ROIs were selected.

Spectra derived from the defined histological ROIs, which were designated as benign, stroma or cancer by a pathologist based on the histomorphology of the HE stained sections, were used to identify two class prediction biomarker candidates. Spectra of defined ROIs - all together 25000 spectra - were extracted, quality checked, normalized and aligned using the internal peaks m/z 4971 and m/z 9256. Logistic Regression (Zhu and Hestie Penalized Logistic Regression, Biostatistics 5:427-443, 2004) was used to classify benign versus cancer, benign versus stroma and cancer versus stroma in order Logistic Regression was used to classify benign versus cancer, benign versus stroma and cancer versus stroma to to identify candidate masses (m/z) that discriminate best between the predefined classes.

The peak with a m/z value associated to a BVR B fragment distinctively distinguished between benign versus cancer as shown in Table 4 and between stroma versus cancer as shown in Table 5.

| Tab. 4 Discriminating peak: benign (reference class) versus cancer | | | |
|---|---|---|---|
| m/z | mean AUC (standard error) | mean sensitivity (standard error) | mean specificity (standard error) |
| 6657.5 | 0.85 (0.076) | 0.65 (0.15) | 0.75 (0.134) |

| Tab. 5 Discriminating peaks: tumor versus stroma (reference class) | | | |
|---|---|---|---|
| m/z | mean AUC (standard error) | mean sensitivity (standard error) | mean specificity (standard error) |
| 6657.5 | 0.9 (0.1) | 0.95 (0.05) | 0.8 (0.111) |

The area under the receiver-operator curve (AUC), sensitivity and specificity were calculated using a 10-fold cross-validation strategy. Highest discriminatory ability was found for mass peaks at m/z 6657.5 (peptide sequnce SEQ ID NO: 2 AIFGATGQTGLTT-LA, flavin reductase, biliverdin reductase B) overexpressed in cancer tissue.

### Example 11: Immunohistochemistry

Immunohistochemistry (IHC) was performed on a Ventana Discovery-XT staining automat (Roche, Basel Switzerland) as described (Bu et al. (2011) Prostate 71, 575-587). First, IHC validation was performed on paraffin embedded tissue of the MALDI-TOF MS tissue imaging patient cohort. 10 cases of low grade prostate cancer (Gleason Score ≤ 3+4) and 10 cases of high grade prostate cancer (Gleason ≥ 4+3) were included. For determination of the expression pattern of biliverdin reductase (BLVR) the following 3 polyclonal rabbit antibodies were used in antibody dilution buffer (Roche), with no antigen retrieval pre-treatment and 60 min of incubation: 1. MyBiosource, no subtype specificity information (MBS194477, 1:50, c = 20 µg/ml, MyBiosource, EMELCA, Netherlands), 2. Sigma, subtype A (BLVRA) specific, (HPA042865, 1:50, c = 1.8 µg/ml, Sigma); 3. Sigma, subtype B (BLVRB) specific, (HPA041937, 1:50, c = 4.4µg/ml, Sigma). For analysis of the expression patterns of MIF, COX8A and TSTD1 the antibodies HPA003868 (Sigma), 15368-1-AP (Proteintech, Manchester, UK) and HPA006655 (Sigma, respectively, were employed. For statistical analysis of biliverdin reductase expression a tissue microarray containing cores of 94 prostate cancer cases (3 tumor and 1 benign cores of each case) was used and stained with the MyBiosource antibody. immunoreactivity was scored using a 3 point scale (0, no staining, 1, weak, 2, moderate, 3, strong staining, (Detre et al. (1995) Journal of clinical pathology 48, 876-878).

### Example 12: Immunohistochemical validation

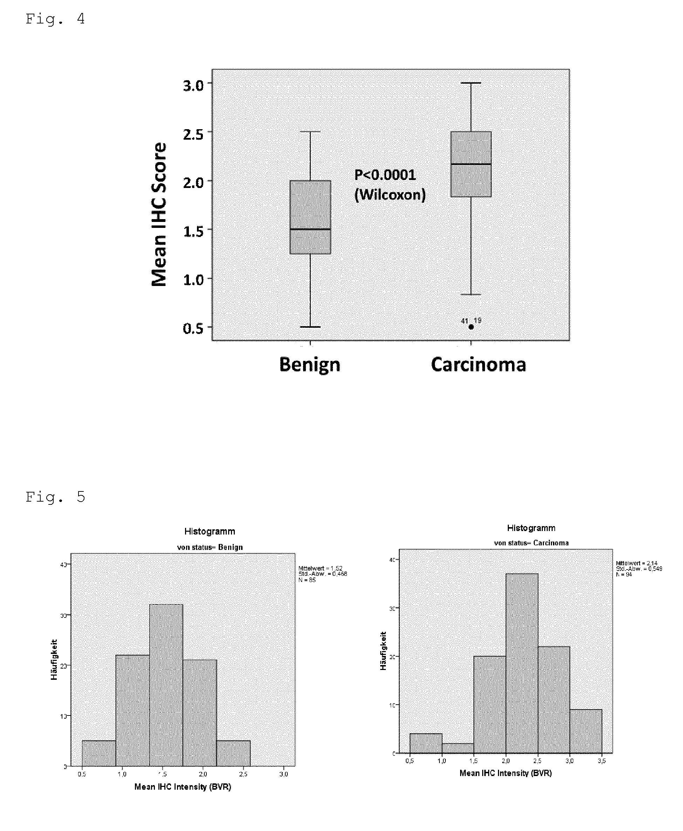

The tissue expression and distribution pattern of biliverdin reductase assessed by IHC mirrored the pattern of the associated mass peak m/z = 6657.5 of the MALDI-MS tissue imaging analysis. Biliverdin immunoreactivity was intense and uniform in most cancer cells, whereas it was weak or absent in benign epithelial cells (Fig. 1). The IHC validation was performed on paraffin embedded tissue sections including several of patients of the MALDI imaging screening cohort. In 10 cases of low grade PCa (Gleason Score ≤ 7 (3+4)) and 10 cases of high grade PCa (Gleason ≥ 7 (4+3)) were included (n=20). Immunoreactivity was scored from 0-3 separately for benign and cancer in each case. The mean score of all cancer tissues was 1.5 compared to a score of 0.9 for benign tissues. Low and high grade cancers differed only little (low grade Ca score 1.6, high grade 1.4). For a thorough statistical analysis of BLVR expression a tissue microarray (TMA) containing 364 tissue cores of 91 prostate cancer specimens (3 tumor and 1 benign cores in each case) was analyzed. After immunostaining each tissue core was scored and assigned to the histological groups. Statistical analysis revealed a highly significant difference between tumor and benign tissue (mean: 1.52 versus 2.14, median 1.50 versus 2.17, p<0.0001 (Wilcoxson)) (Fig. 4).

There are two subtypes of the protein, A (encoded by BLVRA) and B (encoded by BLVRB). In the protein identification analysis a fragment of BLVRB was identified and assigned to a discriminatory m/z peak. In order to assess which biliverdin reductase subtype is expressed in malignant and non-malignant prostate, two polyclonal rabbit antibodies specific for subtypes A and B provided by the human protein atlas consortium were employed. As can be seen in Fig. 1C and D, both subtypes are expressed in prostate cancer cells and both show a very similar pattern and tumor to benign distribution.

In conclusion the IHC validation studies confirmed that the expression pattern of biliverdin reductase reflects the tissue distribution of the benign/cancer discriminatory m/z mass to which this protein was assigned in the protein identification analysis. These data suggest biliverdin reductase as a biomarker for prostate cancer.

### Example 13: mRNA assay for BVR B in urine samples in combination with PSA

Total RNA was extracted from urine exosomes (small vesicles containing cellular components, secreted by cells; prostate epithelial cells are active producers of exosomes; in the older literature also described as "prostasomes"), transcribed to cDNA. MRNA fragments for housekeeping genes RHOA, TBP, for the prostate specific gene PSA and for BLVRB were amplified using a two step amplification method (combinded preamplification of target genes using a mixture of specific primer, followed by a real-time PCR amplification. Samples: Benign, 13 samples; Low Gleason, 8 samples; High Gleason, 10 samples).

BLVRB level normalized to house keeping genes (combined TBP and RHOA1) showed no discriminatory power between benign and PCa or low Gleason and high Gleason tumors. Only samples with measurable marker and measurable PSA were included.

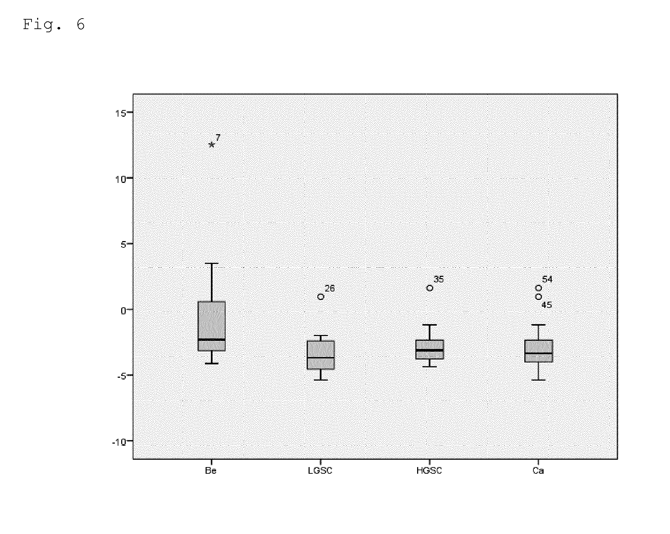

When BLVRB was related to PSA, assuming that PSA level is a surrogate marker for the amount of mRNA originating in the prostate, the differences between benign and PCa groups were of statistical significance (Fig 6).

The ratio BLVRB/PSA BLVRB was lower in the urine exosomes of PCa patients compared with benign patients.

Prostate exudate enrichment, e.g. by enrichment of urinary exosomes, can further increase BVLR A and B diagnostic significance. Both BVLR A and B mRNA and protein can be significantly detected after enrichment, which allows a diagnosis with BVLR as exclusive marker. Prostate derived exosomes can e.g. be enriched by immunoaffinity purification. Similar procedures can be used on serum samples.

## Claims

1. Use of biliverdin reductase (BVR), preferably BVR B, as protein marker for diagnosis of prostate cancer in a tissue sample or in a body fluid sample.

2. Method for diagnosing prostate cancer in a patient comprising the following steps:
- providing a tissue sample or a body fluid sample of said patient;
- determining the amount of BVR proteins in the sample; and
- comparing the amount of BVR proteins in the sample with a sample of known prostate cancer status.

3. Method of detecting prostate cancerous cells in a sample of a patient comprising:
- providing a tissue sample or a body fluid sample of said patient;
- determining the amount of BVR proteins in the sample; and
- comparing the amount of BVR proteins in the sample with a sample of known prostate cancer status.

4. The method of claim 2 or 3, wherein the sample of known prostate cancer status is of a healthy subject or a subject with prostate cancer.

5. Method according to claim 2, 3 or 4, **characterized in that** the amount of BVR proteins is determined by immunohistochemical (IHC) methods, by immunofluorescence (IF) methods, by mass-spectrometry (MS), by imaging mass spectrometry (IMS) or by a combination of these methods.

6. Method according to claim 5, **characterized in that** the determination is by MALDI-MS, preferably MALDI-IMS.

7. Method according to claim 5 or 6, **characterized in that** the determination is by identifying a peptide fragment with an m/z ratio of between 6648 and 6668, preferably of 6657, by mass spectrometry.

8. Method according to any one of claims 2 to 7, **characterized in that** the sample is a prostate tissue sample, a sample derived from blood, a saliva sample, a bladder washing sample, a semen sample or a urine sample, especially a prostate biopsy or a plasma or serum sample.

9. Use of a kit for detecting the amount of BVR proteins in a sample, comprising specific detection agents for amount of BVR proteins or cellular degradation products thereof for diagnosing a tissue sample or a sample of a body fluid for prostate cancer.

10. Use or method according to any one of claims 1 to 9, wherein BVR is BVR B or BVR A or a combination of BVR A and BVR B.

11. Method of diagnosing prostate cancer in a subject comprising determining BVR, preferably BVR B, in a sample of said subject, preferably as defined in claims 1 to 10, in combination with determining at least a second non-BVR prostate cancer marker, preferably selected from PSA (and/or PSA subtypes), PSM (prostate specific membrane antigen), hK2 (human glandular kallikrein 2), AMACR (alpha-methylacyl-CoA racemase), hepsin or PCA3 (PC antigen 3).

12. BVR inhibitor for use in a prostate cancer therapy.

13. Inhibitor for use according to claim 11, wherein said inhibitor is a BVR B inhibitor, preferably used in combination with a BVR A inhibitor.

14. Inhibitor for use according to claim 11 or 12, wherein said inhibitor is an antibody or BVR B binding antibody fragment, preferably adapted for intracellular delivery, or a nucleic acid inhibitor, preferably an antisense inhibitor or siRNA, or nucleic acid inhibitor encoding vector, preferably adapted for intracellular delivery.


SEQUENCE LISTING

<110>  IMG INSTITUT FÜR MEDIZINISCHE GENOMFORSCHUNG PLANUNGSGESELLSCHAFT
M.B.H.

<120>  USe of biliverdin reductase proteins as cancer marker

<130>  r56974

<160>  2

<170>  PatentIn version 3.3

<210>  1
<211>  50
<212>  DNA
<213>  Artificial

<220>
<223>  microarray probe

<400>  1
ggacatgagg agcaaaggaa gggggcaata aatgttgagc caagagcttc          50


<210>  2
<211>  15
<212>  PRT
<213>  Artificial

<220>
<223>  MALDI fragment

<400>  2

Ala Ile Phe Gly Ala Thr Gly Gln Thr Gly Leu Thr Thr Leu Ala
1               5                   10                  15